# EUROPEAN PATENT SPECIFICATION

(11) **EP 0 722 459 B1**
(45) Date of publication and mention of the grant of the patent: **23.12.1998**
(21) Application number: 94920544.7
(22) Date of filing: 11.07.1994
(51) Int. Cl.: C07K 7/06, C07K 5/083, A61K 38/08

(54) **HEMOREGULATORY PENTA- OR HEXAPEPTIDES**
HAEMOREGULATORISCHE PENTA-ODER HEXAPEPTIDE
PENTA/PEPTIDES OU HEXAPEPTIDES HEMOREGULATEURS

(30) Priority: 09.07.1993 GB 9314200
(43) Date of publication of application: 24.07.1996
(73) Proprietor: NYCOMED IMAGING AS, 0401 Oslo 4 (NO); SMITHKLINE BEECHAM CORPORATION, Philadelphia Pennsylvania 19103 (US)
(72) Inventor: AGNER, Erik, N-1187 Oslo (NO); CUTHBERTSON, Alan, N-0861 Oslo (NO); FISCHER, Peter, N-0382 Oslo (NO); BHATNAGAR, Pradip, Exton, PA 19341 (US); HUSBYN, Mette, N-0861 Oslo (NO); UNDHEIM, Kjell, N-1300 Sandvika (NO)
(74) Representative: Matthews, Derek Peter
(86) International application number: GB9401501
(87) International publication number: WO9501990

(56) References cited:
- EP-A- 0 408 371
- WO-A-88/03535

## Description

The present invention relates to the use of peptides having a stimulating effect on cell proliferation, and to novel peptides having specific and/or general cell stimulating effects.

The mammalian body contains cells having enormously diverse structures and functions, and the mechanisms of differentiation and development have been the focus of much study. It is known that for systems of cells having a continuous turnover the mechanism commonly involves a reservoir of pluripotent stem cells which divide and constantly supply new cells to the system. While initially homogeneous the stem cells supplied from the "reservoir" soon become committed to one or other morphology and subsequently develop into the required functional cells.

Examples of such stem cell systems are the haemopoietic system in bone marrow and the epithelial and epidermal systems.

The manipulation or control of stem cell division is of great potential therapeutically and much research continues to be devoted to elucidating the mechanisms involved and the chemical messengers responsible. To date several biomolecules have been identified as possessing a role in cell production and differentiation either by the stimulation or inhibition of a step within the process. Myelopoiesis has been particularly well studied in this regard and molecules involved in its control include: colony-stimulating factors (CSF) such as granulocyte colony-stimulating factor (G-CSF), macrophage colony-stimulating factor (M-CSF), granulocyte-macrophage colony-stimulating factor (GM-CSF), multi-lineage colony-stimulating factor (multi-CSF; IL-3) [see Metcalf, Science 229: 16 (1985)], interleukin 11 (IL-11) [see Paul et al Proc Natl Acad Sci USA 87: 7521 (1990)], Lactoferrin [see Broxmeyer et al Blood Cells 11: 429 (1986)], prostaglandins [see Pelus et al J. Immunol 140: 479 (1988)], acidic (H-subunit) ferritin [see Broxmeyer et al Blood 68: 1257 (1986)], interferons (α, β and γ) [see Pelus et al supra. and Broxmeyer et al J. Immunol 131: 1300 (1983)], tumour necrosis factors (α and β) [see Broxmeyer et al J Immunol 136: 4487 (1986)], transforming growth factor-β [see Ottman et al J Immunol 140: 2661 (1988)], and activin and inhibin [see Broxmeyer et al Proc Natl Acad Sci USA 86: 779 (1989)].

It has also been found that the haemoregulatory pentapeptide (pEEDCK) inhibits the proliferation of myelopoietic cells selectively [see Paukovits et al Z. Naturforsch 37: 1297 (1982)] and other peptides corresponding to a narrow general formula were discovered to exert a similar inhibitory effect in hemopoiesis [see EP-A-112656 and WO90/02753]. Oxidation of the peptide monomers resulted in dimeric molecules linked by a cysteine bridge and these dimeric molecules were found to stimulate myelopoiesis [see Laerum et al. Exp. Hematol 16: 274 (1988)]. The (pEEDCK)₂ dimer and other similar compounds are disclosed in WO-A-88/03535. Further dimeric peptide compounds are disclosed in EP-A-408371 in which the disulphide bond has been replaced by a carbon or carbon/sulphur bridge linking the selected peptide chains. The bridge is thus relatively stable to hydrolysis but is itself inert and incapable of participating in receptor-dimer interactions.

Whilst we do not wish to be bound by theoretical considerations, it is presently believed that such peptide compounds interact with stromal cells in vivo and that the stromal cells are responsible for stimulating or inhibiting cellular division via other soluble factors. The dimers are thus believed to induce or promote stromatic production of stimulatory cellular regulatory factor(s) whilst the monomeric peptides may either inhibit that process or cause the production of factors which prevent or hinder cell division. Thus, according to current thinking, the stromal cells may act to amplify the stimulatory or inhibitory effects of the dimeric and monomeric peptides respectively.

There is a continuing need for peptide compounds capable of stimulating cell proliferation to a useful level in vivo. In this regard it should be noted that different degrees of stimulation may be more appropriate to certain clinical situations than to others and, in particular, selective stimulation of individual cell types is important.

The present invention is concerned with dimeric peptide compounds in which the individual peptide chains are joined to one another by two or more bridging units, which may be in the form of diaminodicarboxylic acid residues and/or chemical bonds between amino acid side chains. Dimeric peptides of this type known in the art contain only a single bridging unit between the peptide chains. These chains thus remain flexible about the bridging unit so that relative inter-chain movement and rotation about the bridge is possible.

By contrast, the peptide compounds of the present invention are conformationally constrained in that the movement of the two peptide chains relative to each other is severely restricted. Surprisingly, it was found that introduction of such steric constraints into haemoregulatory peptides did not destroy the desired biological activity, ie. stimulation of cell proliferation, despite the fact that substantial structural and functional modification of both peptide backbone and amino acid side chains had been performed. Furthermore, it was found that both pseudo-parallel as well as pseudo-anti-parallel dimeric peptides were able to exert a stimulating effect on cell proliferation.

The term "pseudo-parallel" is used herein to mean that the long axis of the peptide chains are held in the same orientation as each other. Thus the amino (and carboxy) termini of the two peptides will be located at the same end of the dimeric peptide.

The term "pseudo-anti-parallel" is used herein to mean that the long axis of the peptide chains are held in opposite orientation to each other. The amino (and carboxy) termini of the two peptide chains are therefore located at opposite ends of the dimeric peptide.

In its broadest aspect, the invention concerns peptide compounds comprising two monomeric peptides having cell proliferation regulatory activity joined together by two or more divalent bridging units. In general, it is preferred that in such a structure, the carboxyl termini of the two peptide chains are at the same end.

Viewed from one aspect, the invention provides peptide compounds in which the monomeric peptides are pseudo-parallel peptides joined by two bridging units terminally attached to the Cα atoms of terminal or non-terminal amino acids in each peptide chain.

Viewed from a further aspect, the invention provides peptide compounds in which the monomeric peptides are pseudo-anti-parallel peptides joined by two or more bridging units terminally attached to the Cα atoms of terminal or non-terminal amino acids in each peptide chain. In these compounds it is preferred that at least one of the bridging units comprises linked amino acid side chains.

particularly preferred peptide compounds in accordance with the invention include those of formulae I, II and III: 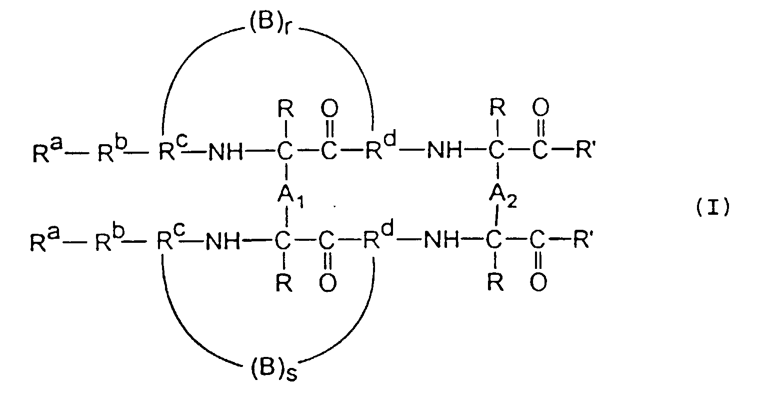 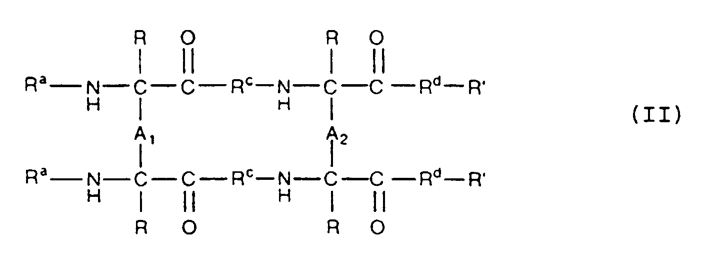 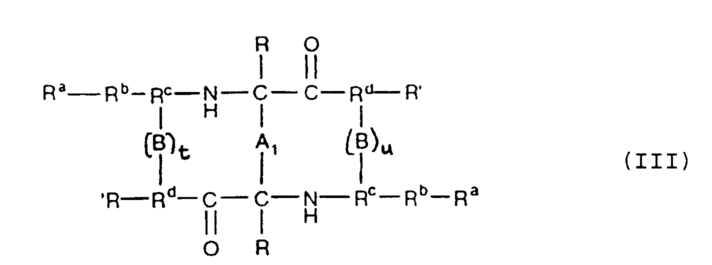 wherein
A₁ and A₂, which may be the same or different, represent
   -(CH₂)ₘ- where m is 0 to 6,
   -CH₂-S-S-CH₂-
   -CH₂-CH=CH-CH₂-,
   -CH₂-C≡C-CH₂-, 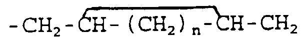 where n is 1 to 4, or 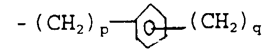 where p and q may each be 0 or 1;
each R^{a} may independently be pyroglutamic acid (p-Glu), picolinic acid (Pic), 3-hydroxypicolinic acid (30H-Pic), proline, N-acetyl-proline, pyrazinecarboxylic acid, pyrazinedicarboxylic acid, nicotinic acid, isonicotinic acid, pipecolinic acid, pyrrolecarboxylic acid, anthranilic acid, quinolinecarboxylic acid, isoquinolinecarboxylic acid, furancarboxylic acid, orotic acid, or pyrazolecarboxylic acid;
each R^{b} may independently be serine (Ser), glutamic acid (Glu), aspartic acid (Asp), threonine (Thr) or allothreonine (aThr);
each R^{c} may independently be aspartic acid (Asp), glutamic acid (Glu) or cysteine (Cys);
each R^{d} may independently be lysine (Lys), ornithine (Orn) or cysteine (Cys);
r and s may each be 0 or 1;
t and u may each be 0 or 1 but at least one of t and u must be 1;
each group B represents the linked side chains of R^{c} and R^{d} such that
   (a) each group B may independently represent -(CH₂)ᵥ-CONH-(CH₂)_{w}-, where v is 1 or 2 and w is 3 or 4, provided that in the peptide chain to which group B is attached R^{c} is Asp or Glu and R^{d} is Lys or Orn; or
   (b) or, each group B may independently represent -CH₂-S-S-CH₂-, where R^{c} and R^{d} of the peptide chain to which group B is attached both represent Cys;
each R may be independently hydrogen or methyl; and
each R' may be a group -OH or -NH₂, and the salts thereof.

In the peptide compounds of the present invention all of the amino acid residues (R^{a}, R^{b} etc.) may be in either the D or L form. The L-form is, however, preferred. Unless otherwise indicated, a symbol such as R^{a} includes the NH and CO groupings attached to the α-carbon atom of the amino acid.

Each end of the bridging groups A₁ and A₂ may have the stereochemistry (R,R), (S,S) or (R,S) at the point of attachment to each peptide chain.

It is preferred that in each of the peptide chains equivalent residues have the same meaning. For example, it is preferred that R^{d} in each peptide chain is Lys and that both groups R^{a}-R^{b}-R^{c}- in formulae I and III are pGlu-Glu-Asp- or Pic-Ser-Asp-.

Likewise, it has been found to be advantageous if each of the bridging moieties present has the same number of carbon atoms separating the two peptide chains. Improved results may be obtained where an even number of carbon atoms is employed in each bridge to separate the peptide chains. Ethylene and butylene bridges are convenient in this regard.

Thus, for example, the following bridge arrangements are particularly suitable in compounds of the present invention:

| Bridge A₁ | Bridge A₂ |
|---|---|
| butylene | butylene |
| butylene | ethylene |
| ethylene | butylene |
| ethylene | ethylene |

Particularly preferred peptide compounds according to the invention are those of formula IV: 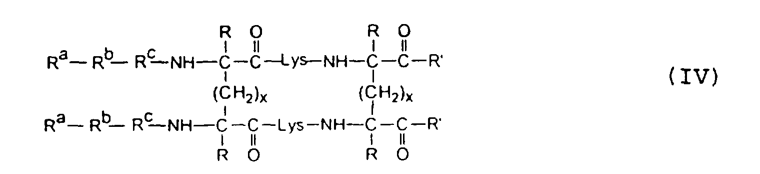 where both symbols x are either 2 or 4;
both groups R^{a}-R^{b}-R^{c}- are either pGlu-Glu-Asp- or Pic-Ser-Asp-, and each R¹ is a group -OH or -NH₂;
and salts thereof.

Compounds of formula IV wherein x is 4 are especially preferred.

Further particularly preferred peptide compounds according to the invention are those of formula V and VI:

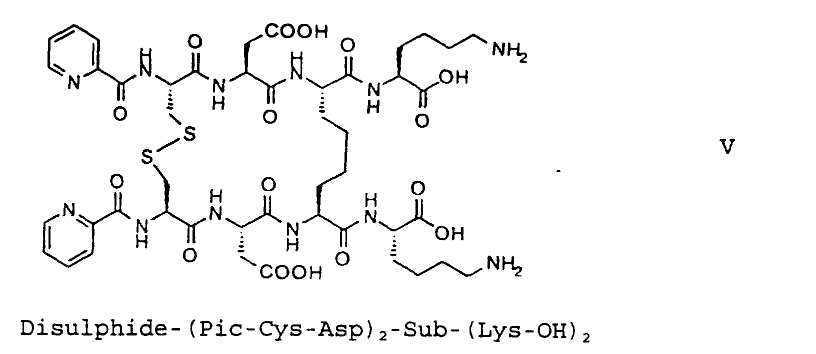

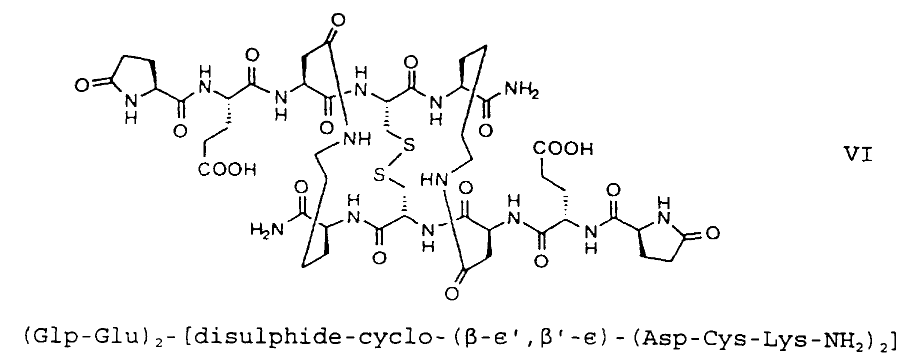

The invention is of particular application in stimulating myelopoiesis in patients suffering from reduced myelopoietic activity, including bone marrow damage, agranulocytosis and aplastic anaemia. This includes treatment of patients having depressed bone marrow function due to immunosuppressive treatment to suppress tissue reactions, e.g. in bone marrow transplant surgery.

The compounds may also be used to promote more rapid regeneration of bone marrow after cytostatic chemotherapy and radiation therapy for neoplastic and viral diseases.

In addition, the new compounds may be of particular value where patients have serious infections due to lack of immune response following bone marrow failure.

Another clinical application will be in combination with the corresponding monomers or related myelopoiesis inhibitors as disclosed in EP-A-112656 or WO-A-90/02753 to induce alternating peaks of high and low activity in the bone marrow cells, thus augmenting the natural circadian rhythm of haemopoiesis. In this way, cytostatic therapy can be given at periods of low bone marrow activity, thus reducing the risk of bone marrow damage, while regeneration will be promoted by the succeeding peak of activity.

In general, in order to exert a stimulatory effect, the peptides of the invention may be administered to human patients orally or by injection in the dose range 0.001-100 mg, for example 1-5 mg, per 70 kg body weight per day. If administered intravenously or subcutaneously, the dose may be in the range 1-10 mg per 70 kg body weight per day, for example about 6 mg, for up to ten days. Nasal, topical (transdermal) or rectal administration is, of course, also feasible. In principle it is desirable to produce a concentration of the peptide of about 10⁻¹³M to 10⁻⁵M in the extracellular fluid of the patient.

According to a further feature of the present invention there are provided pharmaceutical compositions comprising as active ingredient one or more peptide compounds according to the invention, especially those compounds of formula (I), (II) and (III) above, or physiologically compatible salts thereof, in association with a pharmaceutical carrier or excipient. The compositions according to the invention may be presented, for example, in a form suitable for oral, nasal, parenteral or rectal administration.

As used herein, the term "pharmaceutical" includes veterinary applications of the invention.

The compounds according to the invention may be presented in the conventional pharmacological forms of administration, such as tablets, coated tablets, nasal sprays, solutions, emulsions, powders, capsules or sustained release forms. Conventional pharmaceutical excipients as well as the usual methods of production may be employed for the preparation of these forms. Tablets may be produced, for example, by mixing the active ingredient or ingredients with known excipients, such as for example with diluents, such as calcium carbonate, calcium phosphate or lactose, disintegrants such as corn starch or alginic acid, binders such as starch or gelatin, lubricants such as magnesium stearate or talcum, and/or agents for obtaining sustained release, such as carboxypolymethylene, carboxymethyl cellulose, cellulose acetate phthalate, or polyvinylacetate.

The tablets may if desired consist of several layers. Coated tablets may be produced by coating cores, obtained in a similar manner to the tablets, with agents commonly used for tablet coatings, for example, polyvinyl pyrrolidone or shellac, gum arabic, talcum, titanium dioxide or sugar. In order to obtain sustained release or to avoid incompatibilities, the core may consist of several layers too. The tablet-coat may also consist of several layers in order to obtain sustained release, in which case the excipients mentioned above for tablets may be used.

Organ specific carrier systems may also be used.

Injection solutions may, for example, be produced in the conventional manner, such as by the addition of preservation agents, such as p-hydroxybenzoates, or stabilizers, such as EDTA. The solutions are then filled into injection vials or ampoules.

Nasal sprays may be formulated similarly in aqueous solution and packed into spray containers either with an aerosol propellant or provided with means for manual compression. Capsules containing one or several active ingredients may be produced, for example, by mixing the active ingredients with inert carriers, such as lactose or sorbitol, and filling the mixture into gelatin capsules.

Suitable suppositories may, for example, be produced by mixing the active ingredient or active ingredient combinations with the conventional carriers envisaged for this purpose, such as natural fats or polyethyleneglycol or derivatives thereof.

Dosage units containing the compounds of this invention preferably contain 0.1-10mg, for example 1-5mg of the peptide of formula (I) or salt thereof.

According to a still further feature of the present invention there is provided a method of stimulation of cell division, especially myelopoiesis, which comprises administering an effective amount of a pharmaceutical composition as hereinbefore defined to a subject.

A further major use of the new peptides, however, is in the production of material for immunological assay techniques. The peptide may then be covalently attached to a suitable high molecular carrier such as albumin, polylysine or polyproline in order to be injected into antibody-producing animals (e.g. rabbits, guinea pigs or goats). In vitro immunisation techniques may also be used. High specificity antisera are obtained by use of well known absorption techniques, using the high molecular carrier. By introducing radioactivity (³H, ¹²⁵I, ¹⁴C, ³⁵S) into the peptide molecule, a radioimmuno assay can be designed and used for determining the peptide in the different biological fluids such as serum (plasma), urine and cerebrospinal fluid.

The peptides of the invention may be synthesised in any convenient way. Suitable methods for forming the amino acid units are described in, for example, "Synthesis of Optically Active α-Amino Acids" by Robert M. Williams (Pergamon Press, 1989). In general, the reactive side chain groups present (amino, thiol and/or carboxyl) will be protected during the coupling reactions of the overall synthesis but it is possible to leave some side chain groups unprotected (hydroxy groups, imidazole groups, primary amine groups, amine groups in cyclic amino acids like pyroGlu) during the entire synthetic procedure.

The final step will thus be the deprotection of a fully protected or a partly protected derivative of a peptide of the general formula I, II or III and such processes form a further aspect of the invention.

In general, the pseudo-parallel structure of formula I may be built up by reacting a compound of the formula VII 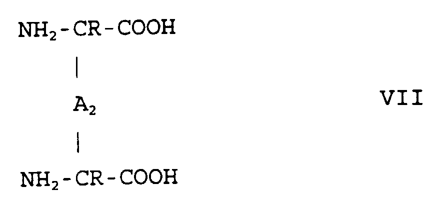 in C-protected (ie. solid-phase bound) form with a N-protected form of acid R^{d} to form a compound of the formula VIII 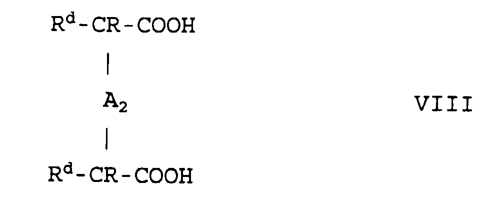 (in N-protected and C-protected form) which may then, after removal of the N-protecting groups, be reacted with a compound of the formula IX 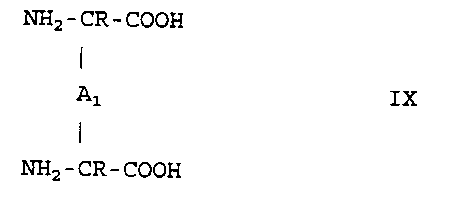 in N-protected form to produce a compound of the formula X 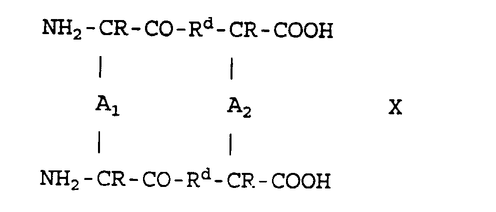 in N- and C-protected form; the latter compound may then be used to build up the N-terminal sequences R^{a}-R^{b}-R^{c} in conventional manner.

An analogous procedure can be used to prepare compounds of formula II.

In order to prepare compounds of formula I containing linked side chains of R^{c} and R^{d}, a compound of formula XI may conveniently be used as a starting material:

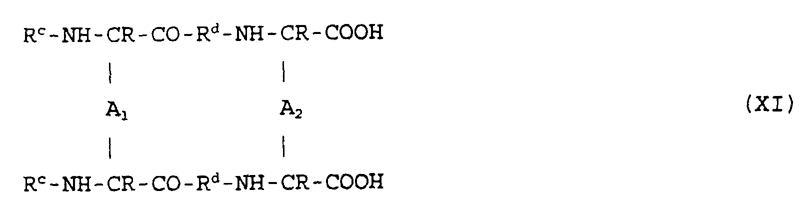

Condensation of the deprotected functional groups of the amino acid side chains of R^{c} and R^{d}, for example formation of an amide bond between side-chain carboxyl and amine function if R^{c} and R^{d} are Asp and Lys respectively, or a disulphide bond if R^{c} and R^{d} are both Cys, will result predominantly in the intra-chain bridges B as shown in Formula XII:

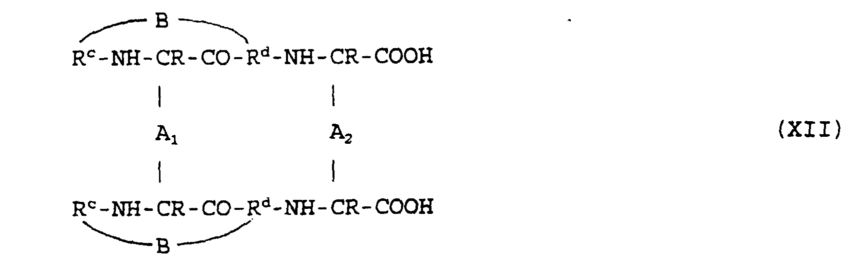

After introduction of amino acid residues R^{a} and R^{b}, the product will be a compound of formula I with both r and s equal to 1.

Compounds of formula XII may be formed from compounds of formula X by adding N-terminal groups R^{c} by the methods described herein.

In order to prepare compounds of formula III containing linked side chains of R^{c} and R^{d}, a compound of formula VIII may conveniently be used as a starting material. The amino acids R^{c} are then introduced using conventional techniques to give a compound of the formula XIII

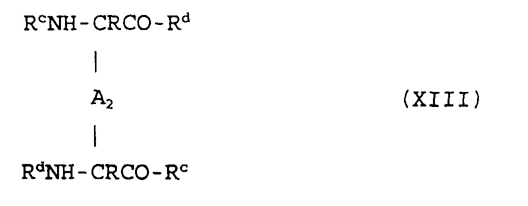

Reaction of this compound with a suitable condensing agent results preferentially in the inter-chain bridges B as shown in formula XIV 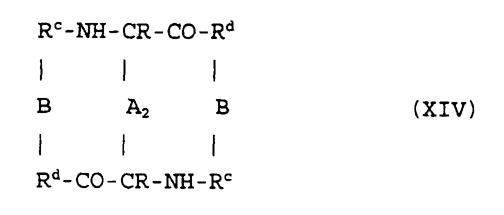 Compound XIV may be reacted with further amino acids R^{a} and R^{b} to build up a compound of formula III. Where A₂ is -CH₂-S-S-CH₂- it may be preferable to reduce the S-S bond and protect the resulting SH groups prior to introducing R^{a} and R^{b}, after which introduction the S-protecting groups may be removed and the S-S- bond reformed by oxidation.

The pseudo-anti-parallel structures of formula III in which A₂ is -CH₂-S-S-CH₂- may be synthesised as summarised in the scheme below which illustrates the formation of the peptide (Glp-Glu)₂-[disulphide-cyclo-(β-ε',β'-ε)-(Asp-Cys-Lys-NH₂)₂] (5)

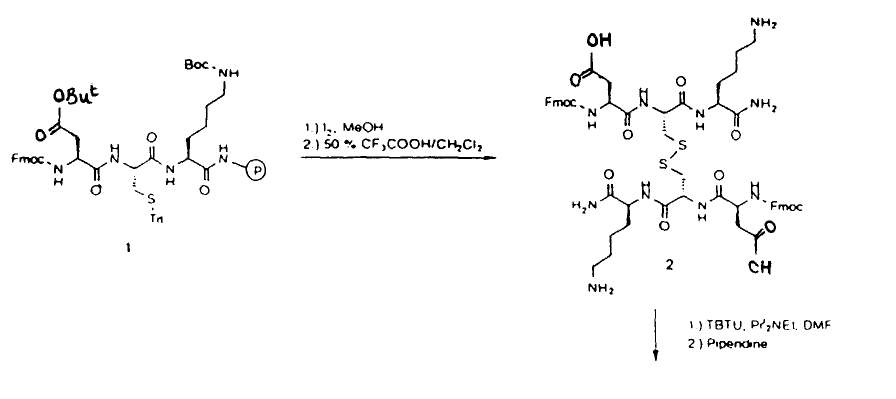

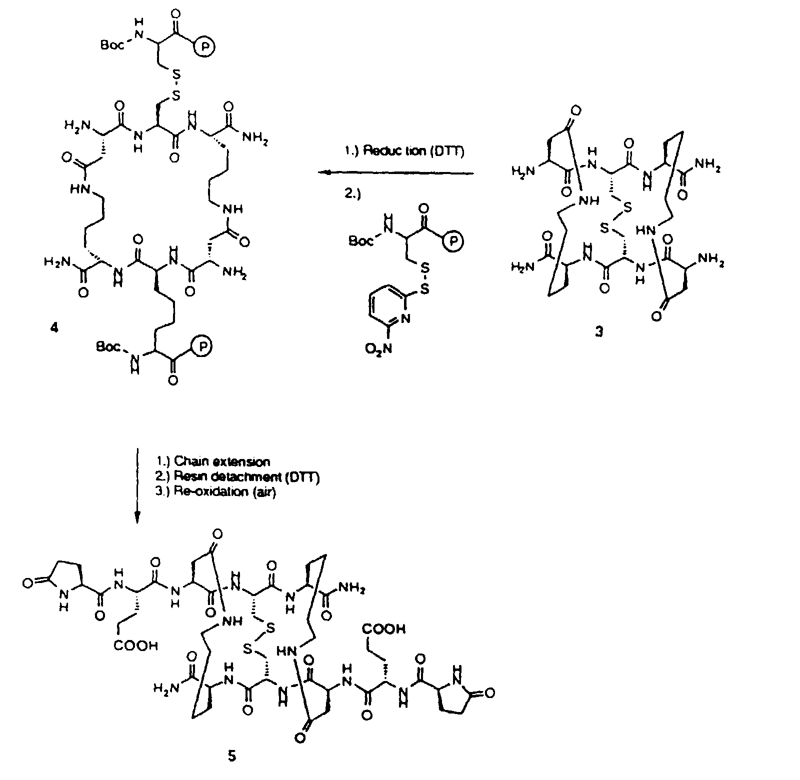

The tripeptide core 1 is assembled on a solid phase using a linker giving rise to a C-terminal carboxamide upon acid cleavage. The cysteine side chain is conveniently protected as the trityl thioether. Upon complete assembly, trityl deprotection and concomitant cystine formation are effected on-resin by treatment with iodine. The dimeric core peptide 2 is then obtained upon simultaneous side-chain deprotection and resin detachment by treatment with trifluoroacetic acid. Further treatment with a condensing agent results in the formation of amide bonds between the Asp and Lys side chains of the two opposing peptide chains. The N-terminal Fmoc groups are then removed to yield the structure 3. The disulphide bond in this compound is then reduced and the peptide is attached to resin-bound BocCys(Npys)-OH through disulphide exchange. The resulting complex 4 is further elaborated N-terminally using Fmoc solid-phase peptide synthesis methods to the desired sequence. Acid-labile side-chain protecting groups are then removed and the peptide liberated from the solid phase by reduction of the disulphide bonds with DTT. The desired peptide 5 is obtained after air oxidation under high dilution.

The compounds VII and IX may be synthesised as described below:

Schöllkopf et al have described the preparation of a variety of amino acids by the metallation and subsequent alkylation of bis-lactim ethers (see, for example, Tetrahedron 39: 2085 (1983) and Topics Curr Chem 109: 65 (1983)). An adaptation of this method has proved particularly useful for the preparation of the bridged amino acids which form part of the present invention. In particular, a bis-lactim ether derived from a valine-glycine dipeptide forms a useful starting compound for the bridging reaction which may be summarized as follows: 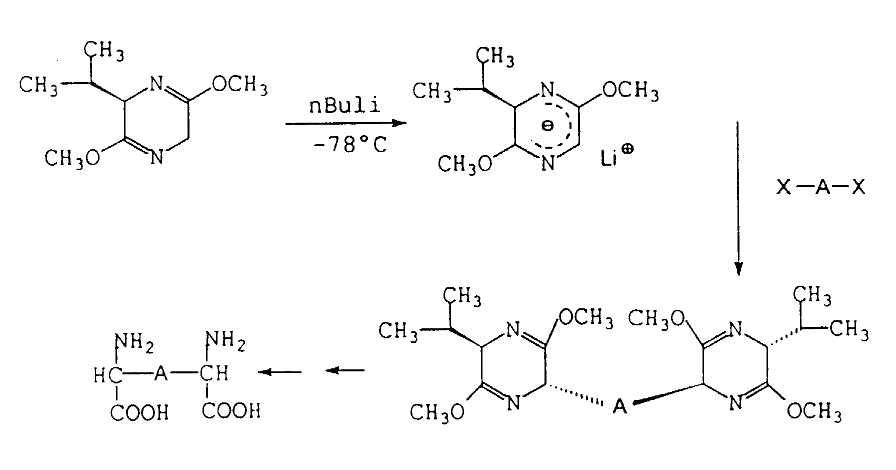 (wherein X is a leaving group such as a halogen atom, for example bromine).

Bridged (S,S)-α,α'-diamino acids can be prepared by this method if D-valine is initially used to form the bis-lactim ether. Equally, bridged (R,R)-α,α'-diamino acids may be formed by the use of L-valine.

We have also found that aryl (including heteroaryl) diglycine derivatives can be prepared from corresponding di-(halocarbonylmethyl) aryl compounds. The latter compounds may be reacted with a chiral oxazolidone to form a carboximide enolate which is then reacted with an azide forming reagent such as 2,4,6-triisopropyl benzenesulphonylazide to introduce azido groups, followed by hydrogenolysis eg. with 10% palladium/charcoal, to produce the desired diglycine.

Our procedure is an adaption of methodology described by Evans et al., in J. Am. Chem. Soc. 109 6881 (1987) and J. Am. Chem. Soc. 111 1063 (1987). Alternatively, a suitable electrophilic amino nitrogen can be found in an azodicarboxylate ester in which case the corresponding dihydrazino acid is an intermediate for hydrogenolysis to the amino acid.

This methodology is illustrated by the reaction scheme given below:

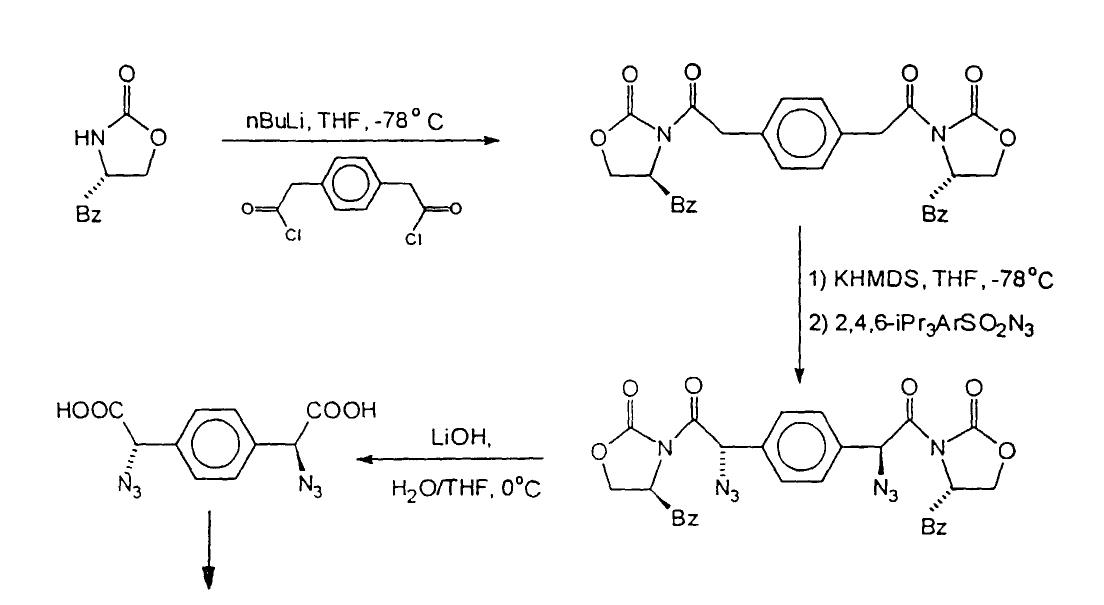

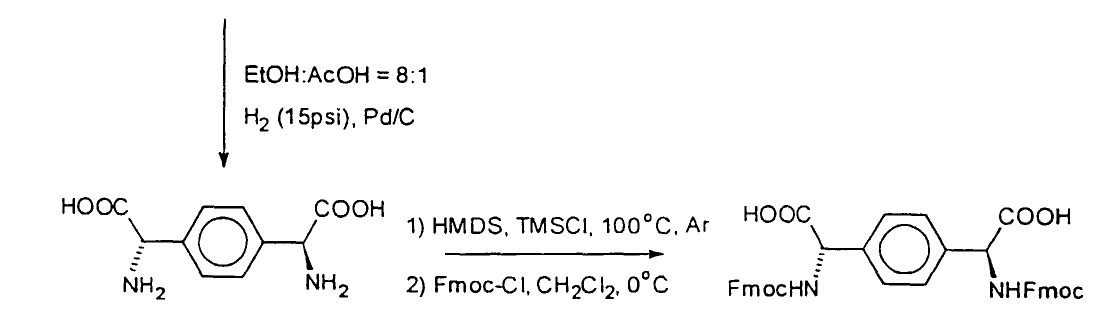

Further the invention also provides a process for producing a peptide compound of formula I, said process comprising
a) metallating and subsequently alkylating a bis-lactim ether to form a bis-lactim dipeptide ether;
b) hydrolysing the bis-lactim dipeptide ether of step (a) to form a bridged α,α'-diamino acid;
c) introducing the remaining amino acids including the other bridged amino acid in the peptide chains; and
d) deprotecting any protected group.

Once the doubly bridged dipeptide has been formed, then the remaining amino acids in the peptide chain can be introduced using conventional techniques.

In building up the peptide chains, one can in principle start either at the C-terminal or the N-terminal although only the C-terminal starting procedure is in common use.

Thus, one can start at the C-terminal by reaction of a suitably protected derivative of, for example lysine with a suitable protected derivative of cysteine. The lysine derivative will have a free α-amino group while the other reactant will have either a free or activated carboxyl group and a protected amino group. After coupling, the intermediate may be purified for example by chromatography, and then selectively N-deprotected to permit addition of a further N-protected and free or activated amino acid residue. This procedure is continued until the required amino acid sequence is completed.

Carboxylic acid activating substituents which may, for example, be employed include symmetrical or mixed anhydrides, or activated esters such as for example p-nitrophenyl ester, 2,4,5,trichlorophenylester, N-hydroxybenzotriazole ester (OBt), N-hydroxysuccinimidylester (OSu) or pentafluorophenylester (OPFP).

The coupling of free amino and carboxyl groups may, for example, be effected using dicyclohexylcarbodiimide (DCC). Another coupling agent which may, for example, be employed is N-ethoxycarbonyl-2-ethoxy-1,2-dihydroquinoline (EEDQ).

In general it is convenient to effect the coupling reactions at low temperatures, for example, -20°C up to ambient temperature, conveniently in a suitable solvent system, for example, tetrahydrofuran, dioxan, dimethylformamide, methylene chloride or a mixture of these solvents.

It may be more convenient to carry out the synthesis on a solid phase resin support. Chloromethylated polystyrene (cross-linked with 1% divinyl benzene) is one useful type of support; in this case the synthesis will start the C-terminal, for example by coupling N-protected lysine to the support.

A number of suitable solid phase techniques are described by Eric Atherton, Christopher J. Logan, and Robert C. Sheppard, J. Chem. Soc. Perkin I, 538-46 (1981); James P. Tam, Foe S. Tjoeng, and R. B, Merrifield J. Am. Chem. Soc. 102, 6117-27 (1980); James P. Tam, Richard D. Dimarchi and R. B. Merrifield Int. J. Peptide Protein Res 16 412-25 (1980); Manfred Mutter and Dieter Bellof, Helvetica Chimica Acta 67 2009-16 (1984).

It is also possible for the coupling reactions to be performed in solution.

A wide choice of protecting groups for amino acids are known and are exemplified in Schröder, E., and Lübke, K., The Peptides, Vols. 1 and 2, Academic Press, New York and London, 1965 and 1966; Pettit, G.R., Synthetic Peptides, Vols. 1-4, Van Nostrand, Reinhold, New York 1970, 1971, 1975 and 1976; Houben-Weyl, Methoden der Organischen Chemie, Synthese von Peptiden, Band 15, Georg Thieme Verlag Stuttgart, NY, 1983; The Peptides, Analysis, synthesis, biology 1-7, Ed: Erhard Gross, Johannes Meienhofer, Academic Press, NY, San Fransisco, London; Solid phase peptide synthesis 2nd ed., John M. Stewart, Janis D. Young, Pierce Chemical Company.

Thus, for example amine protecting groups which may be employed include protecting groups such as carbobenzoxy (Z-), t-butoxycarbonyl (Boc-), 4-methoxy-2,3,6-trimethylbenzene sulphonyl (Mtr-), and 9-fluorenylmethoxycarbonyl (Fmoc-). It will be appreciated that when the peptide is built up from the C-terminal end, an amine protecting group will be present on the α-amino group of each new residue added and will need to be removed selectively prior to the next coupling step. For solid phase systems one particularly useful group for such temporary amine protection is the Fmoc group which can be removed selectively by treatment with piperidine in an organic solvent. For synthesis in solution, Boc- is a preferred protecting group, which can be introduced and removed in a conventional manner.

The amino acids or peptides often require to be silylated prior to protection eg. by addition of Fmoc in order to improve their solubility in organic solvents. Silylation and Fmoc protection reactions are summarized below: 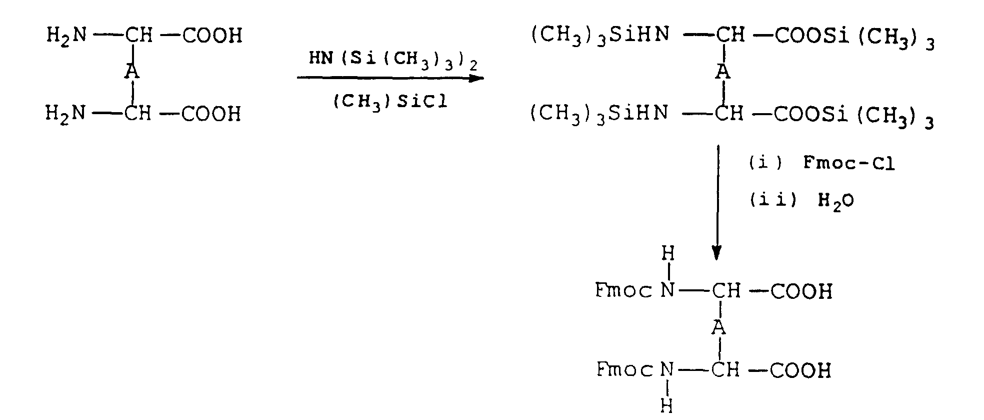 Carboxyl protecting groups which may, for example be employed include readily cleaved ester groups such as benzyl (-OBZl), p-nitrobenzyl (-ONB), or t-butyl (-tOBu) as well as the coupling on solid supports, for example methyl groups linked to polystyrene.

Thiol protecting groups include p-methoxybenzyl (Mob), trityl (Trt) and acetamidomethyl (Acm).

It will be appreciated that a wide range of other such groups exists as, for example, detailed in the above-mentioned literature references, and the use of all such groups in the hereinbefore described processes fall within the scope of the present invention.

A wide range of procedures exists for removing amine- and carboxyl-protecting groups. These must, however, be consistent with the synthetic strategy employed. The side chain protecting groups must be stable to the conditions used to remove the temporary α-amino protecting groups prior to the next coupling step.

Amine protecting groups such as Boc and carboxyl protecting groups such as tOBu may be removed simultaneously by acid treatment, for example with trifluoro acetic acid. Thiol protecting groups such as Trt may be removed selectively using an oxidation agent such as iodine.

The cystein containing peptides may be synthesised by the methods described in the text with removal of all protecting groups including the thiol protecting groups as the last synthetic step.

The following Examples are given by way of illustration only.
The following abbreviations are used in the Examples:
- Asp =: aspartic acid
- But =: tert. butyl
- Ser =: serine
- Glu =: glutamic acid
- pGlu =: pyroglutamic acid
- PyBOP =: (benzotriazolyloxy-tris [pyrrolidino]phosphonium hexafluorophosphate
- HOBt =: 1-hydroxybenzotriazole
- NMM =: 4-methylmorpholine
- DMF =: dimethylformamide
- DMAP =: N,N-dimethylaminopyridine
- Pic =: 2-picolinic acid
- Boc =: tert. butyloxycarbonyl
- DIC =: diisopropylcarbodiimide
- DCM =: dichloromethane
- TFA =: trifluoroacetic acid
- HPLC =: high performance liquid chromatography
- Adp =: L,L-2,5-Diaminoadipic acid
- Glp =: Pyroglutamic acid
- DTT =: Dithiothreitol
- HSF =: Haematopoietic stimulating factor
- Npys =: 3-Nitro-2-pyridinesulphenyl
- Pim =: L,L-2,6-Diaminopimelic acid
- Sub =: L,L-2,7-Diaminosuberic acid
- TBTU =: 2-(1H-benzotriazol-1-yl)-1,1,3,3-tetramethyluronium tetrafluoroborate

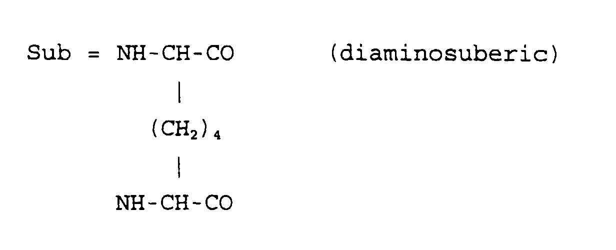

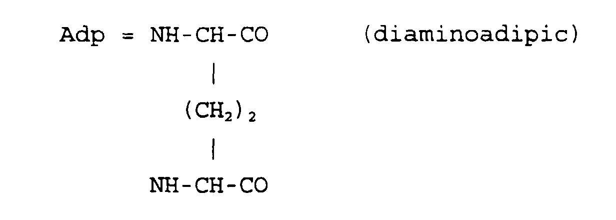

### Example 1

### Preparation of (Pic-Ser-Asp)₂Sub(Lys)₂Sub.OH

Fmoc-Sub-OH (0.65 g, 1 mmol) was first activated with PyBOP (1.04 g, 2 mmol), NMM (0.33 ml, 3 mmol) and HOBt (0.27 g, 2 mmol) in DMF (10 ml) for 2 minutes. The activated amino acid was added to Wang resin (2.0 g, 0.8 mmol/g) already swollen in DMF and then DMAP, dissolved in DMF (1 ml), was added. The mixture was sonicated for 3 hours and then washed well with DMF.

An aliquot of resin (approx. 20 mg) was removed and washed with DCM and diethyl ether before drying overnight in a vacuum dessicator.

A resin loading calculation based on UV detection at 290 nm was performed. The amount of Fmoc liberated from the deprotection of a known weight of dried resin showed a 20% attachment of the first amino acid. This corresponds to an incorporation of 0.16 mmol/g.

Unreacted acid functions were capped by formation of the 2-pyridylethyl ester derivative. The resin (2.0 g, 0.16 mmol/g) was swollen in DMF (20 ml) and DIC (0.15 ml, 1.0 mmol), HOBt (0.034 g, 0.25 mmol) and 2-(2-hydroxyethyl)pyridine (0.34 ml, 3.0 mmol) added at 4°C with gentle stirring. The mixture was stirred at 4°C for a further 2 hours then at ambient temperature for 16 hours. The resin was then transferred to a manual nitrogen bubbling apparatus (see E. Atherton and R. C. Sheppard, "Solid Phase Peptide Synthesis", IRL Press, Oxford, 1989, p 89) where it was washed well with DMF (8 X 10 ml).

The standard protocol for a manual bubbler synthesis is shown in Table 1.

**Table 1**

| | |
|---|---|
| 1. | DMF wash (8 x 1 min) |
| 2. | 20% Piperidine/DMF (1 x 3 min) |
| 3. | 20% Piperidine/DMF (1 x 7 min) |
| 4. | DMF wash (8 x 1 min) |
| 5. | Ninhydrin test |
| 6. | Coupling of activated Fmoc amino acid |
| 7. | DMF wash (8 x 1 min) |
| 8. | Ninhydrin test. If - ve return to step 2. If + ve return to step 6. |

After N^{α} deprotection in the bubbler, Fmoc-Lys(Boc).OH (2.3 g, 4.96 mmol) was coupled as the symmetrical anhydride by addition of DIC (0.38 ml, 2.48 mmol) to a solution of the amino acid in DMF (10 ml). The anhydride was allowed to preform for 10 minutes. The acylation was shown to be complete after 1.5 hours by Kaiser test (see E. Kaiser, R.L Colescott et. al.; Analytical Biochemistry , 1970, 34, p 595).

The (Fmoc-Lys[Boc])2Sub-resin from above (0.5 g, 0.16 mmol/g) was deprotected in the bubbler using standard protocols. Fmoc-Sub-OH (42 mg, 0.065 mmol) preactivated for 10 minutes in DMF (8 ml) containing DIC (0.1 ml, 0.65 mmol) and HOBt (0.088 g, 0.65 mmol) was added and bubbled for 16 hours. Unreacted acid functions were treated with a further portion of DIC and HOBt for 3 hours. After washing with 30% MeOH/DCM and acetylation, the remaining amino acids were coupled using standard manual protocols and excesses detailed in Table 2.

**Table 2**

| | |
|---|---|
| Amino Acid | Fmoc-Asp(OBut)-OH 1 mmol 0.41 g Fmoc-Ser(But)-OH 1 mmol 0.38 g Picolinic acid 1 mmol 0.12 g |
| | |
| Activation | All acids activated with PyBOP (0.52 g, 1 mmol), NMM (0.16 ml, 1.5 mmol) and HOBt (0.14 g, 1.0 mmol) |
| | |
| Procedure | Amino acid, PyBOP and HOBt are dissolved in DMF (5 ml) then NMM added. The mixture is then added to the deprotected resin in the bubbler and coupled until the Kaiser test is - ve. |

The peptide was cleaved from the resin in 5% aqueous TFA for 2 hours before evaporation of the cleavage mixture in vacuo. The crude peptide was liberated as a white solid (83 mg) by addition of diethyl ether.

Purification of the crude peptide (40 mg) by preparative HPLC (vydac TP1002, C18, 20 cm column) using a 5-40% B gradient (B = 40% CH₃CN in water + 0.1% TFA, flow = 9 ml/min) over 40 minutes yielded 3.4 mg of the title compound after lyophilisation; amino acid analysis Asp₂ 2.46, Ser₂ 2.00, Sub₂ 1.85, Lys₂ 2.3, Pic N.D; m/z (FAB) (M+) requires 1242, found 1243; HPLC (vydac 218TP54, C18, 20 cm column, 5-50%B over 20 minutes), Retention time = 17.5 minutes.

### Example 2

### Preparation of (Pic-Ser-Asp)₂Adp(Lys)₂Adp.NH₂

The synthesis was carried out on Rink resin (0.83g, 0.1 mmol; Novabiochem, Cat. NS-62-0024; substition 0.12 mmol/g). Fmoc-Adp-OH (25 mg, 0.04 mmol), PyBOP (42 mg, 0.08 mmol), HOBt (11 mg, 0.08 mmol) and NMM (0.013 ml, 0.12 mmol) in DMF (6 ml) was added to the Na deprotected resin in a 50 ml flask and the reaction allowed to proceed for 16 hours. Additional PyBOP, HOBt and NMM were added and the reaction allowed to proceed for a further 3 hours. The polymer was then transferred to a flow vessel and the remaining residues coupled automatically on a Biolynx peptide synthesiser. Standard protocols were employed using Fmoc-Lys(Boc)-OH (0.23 g, 0.5 mmol), Fmoc-Adp-OH (25 mg, 0.04 mmol), Fmoc-Asp(OBut)-OH (0.2 g, 0.5 mmol), Fmoc-Ser(But)-OH (0.19 g, 0.5 mmol) and 2-Pic.OH (62 mg, 0.5 mmol). Activation was carried out with PyBOP (0.26 g, 0.5 mmol), HOBt (68 mg, 0.5 mmol) and NMM (0.08 ml, 0.75 mmol) with a coupling time of 1 hour for the residues Lys, Asp, Ser and Pic.

Activation of Fmoc-Adp-OH (25 mg, 0.04 mmol) was once again carried out with PyBOP (42 mg, 0.08 mmol), HOBt (11 mg, 0.08 mmol) and NMM (0.013 ml, 0.12 mmol) with a coupling time of 3 hours. Additional PyBOP, HOBt and NMM was added and the reaction allowed to proceed for a further 2 hours.

Cleavage was caried out in 5% aqueous TFA for 1.5 hours. The TFA mixture was evaporated in vacuo and the peptide precipitated with ether. After drying the crude peptide weighed 40 mg.

Purification of the crude peptide (40 mg) by preparative HPLC (vydac TP1002, C18, 20 cm column) using a 5-50% B gradient (B = 40% CH₃CN in water + 0.1% TFA, flow = 10 ml/min) over 35 minutes yielded 2.5 mg of the title compound after lyophilisation; m/z (FAB) (M+) requires 1185, found 1185.7; HPLC (vydac 218TP54, C18, 20 cm column, 5-50%B over 20 minutes), Retention time = 16.8 minutes.

### Examples 3-9

### Preparation of (Waa-Xaa-Asp)₂-Yaa-(Lys)₂-Zaa-R₂

| Example No. | Waa | Xaa | Yaa | Zaa | R₂ | HPLC t_{R} (min) | Formula | FAB-MS [M]⁺ |
|---|---|---|---|---|---|---|---|---|
| 3 | Glp | Glu | Sub | Sub | OH | 16.4^{a} | C₅₆H₈₆N₁₄O₂₄=1339.4 | 1338.6 |
| 4 | Glp | Glu | Adp | Sub | OH | 15.7^{a} | C₅₄H₈₂N₁₄O₂₄=1311.3 | 1311.7 |
| 5 | Pic | Ser | Adp | Sub | OH | 17.4^{b} | C₅₂H₇₄N₁₄O₂₀=1215.2 | 1215.2 |
| 6 | Glp | Glu | Adp | Adp | OH | 13.4^{a} | C₅₂H₇₈N₁₄O₂₄=1283.3 | 1283.2 |
| 7 | Pic | Ser | Sub | Pim | OH | 18.6^{b} | C₅₃H₇₆N₁₄0₂₀=1229.3 | 1229.6 |
| 8 | Pic | Ser | Sub | Sub | NH₂ | 16.5^{b} | C₅₄H₈₀N₁₆0₁₈=1241.3 | 1242.0 |
| 9 | Pic | Ser | Sub | Sub | ^{c} | 13.5^{b} | C₉₈H₁₅₂N₂₄0₂₈S₂=2178.5 | 2180.3 |

| | | | | | | | | |
|---|---|---|---|---|---|---|---|---|
| ^{a}) Vydac 218TP54, 4.6 x 25 cm, 1 mL/min, 0 to 30% CH₃CN in 0.1% aq CF₃COOH over 20 min | | | | | | | | |
| ^{b}) Vydac 218TP54, 4.6 x 25 cm, 1 mL/min, 5 to 50% CH₃CN in 0.1% aq CF₃COOH over 20 min | | | | | | | | |
| ^{c}) Aminocaproate-Lys (Biotin) -OH | | | | | | | | |

These compounds were synthesised analogously to the title compounds of Examples 1 and 2, using Fmoc₂-Adp(OH)₂, Fmoc₂-Pim-(OH)₂ and Fmoc₂-Sub-(OH)₂ at sequence positions 4 and 6. Glp-OH instead of Pic-OH and Fmoc-Ser(Bu^{t})-OH instead of Fmoc-Glu(OBu^{t})-OH at positions 1 and 2, respectively, were used where appropriate.

### Example 10

### Preparation of disulphide-(Pic-Cys-Asp)₂Sub-(Lys-OH)₂

### [Pic-Cys(SBu^{t})-Asp]₂-Sub-(Lys-OH)₂

Fmoc-Lys(Boc)-SASRIN resin (5g; 0.6 mmol/g) was washed with DMF and then treated with 20% piperidine in DMF for 15 min to remove Fmoc groups. After repeated DMF washing, Fmoc₂-Sub-(OH)₂ (638 mg, 1 mmol) was coupled to the deprotected resin with the aid of DCC (1.03 g, 5 mmol) and HOBt (765 mg, 5 mmol) in DMF during 10 h. After washing, a further coupling cycle (1h) was performed with similar quantities of DCC and HOBt in order to couple any remaining free Sub carboxyl groups to resin-bound Lys. The dipeptidyl resin was then washed with 30% MeOH/CH₂Cl₂ (2 x 20 min) and DMF. Unreacted Lys amino groups were capped by reaction with 10% Ac₂O/DMF (2 x 50 mL) during 1 h. After renewed washing cycles with DMF, alternate deprotection and acylation cyles were performed until the title sequence had been produced. Fmoc-Asp(OBu^{t})OH, Fmoc-Cys(SBu^{t})-OH and Pic-OH (3-fold molar excess) were coupled until a negative Kaiser test was recorded with the aid of 1 equivalent each of PyBOP and HOBt, as well as 1.5 equivalents of Prⁱ₂NEt. After complete chain assembly, the peptidyl resin was washed with CH₂Cl₂ and Et₂O, before being dried in vacuo. The resulting material was shaken in CF₃COOH, containing 5% H₂O, for 1 h. Resin residue was then filtered off and washed with neat CF₃COOH. The combined filtrate and washings were rotary evaporated. The residue was triturated with Et₂O, dried, redissolved in 0.1% aq CF₃COOH and lyophilised.

Analytical RP-HPLC (Vydac 218TP54, 0.46 x 25 cm, 1 mL/min, 5 to 75% CH₃CN gradient over 20 min in 0.1% aq CF₃COOH, λ = 215 nm): Retention time = 15.8 min.

### Disulphide-(Pic-Cys-Asp)₂Sub-(Lys-OH)₂

An aliquot of [Pic-Cys(SBu^{t})-Asp]₂-Sub-(Lys-OH)₂ (20 mg) was dissolved in CF₃CH₂OH/H₂O (95:5, 10 mL) and tributylphosphine (3 drops) was added. This solution was shaken for lh, following which it was rotary evaporated. The residue of reduced (Pic-Cys-Asp)₂-Sub-(Lys-OH)₂ was triturated with Et₂O, filtered and dried.

Analytical RP-HPLC (conditions as above for Cys-protected material) showed a Retention time of 10.80 min. A solution of DMSO/CF₃COOH (5:95; 20 mL) was then added and the oxidation reaction allowed to proceed for 75 min. CF₃COOH was then removed by rotary evaporation. The residual peptide solution in DMSO was then applied to an RP-HPLC column (Vydac 218TP1022, 2.2 x 25 cm), which was developed at 5 mL/min with a gradient from 5 to 60% CH₃CN in 0.1% aq CF₃COOH over 2 h. Fractions containing oxidised peptide were pooled and lyophilised to yield 2.0 mg of pure title compound.

Analytical RP-HPLC (conditions as above for Cys-protected material); Retention time = 10.45 min. A co-injection of reduced and oxidised material resulted in base-line separation of the two components.
FAB-MS: [M + H]⁺ = 1105.4;
C₄₆H₆₄N₁₂O₁₆S₂ = 1105.20.

### Example 11

### Preparation of (Glp-Glu)₂-[disulphide-cyclo-(β-ε',β'-ε)-(Asp-Cys-Lys-NH₂)₂]

### Disulphide-cyclo-(β-ε',β',-ε)-(H-Asp-Cys-Lys-NH₂)₂]

The tripeptide Fmoc-Asp(OBu^{t})-Cys(Trt)-Lys(Boc)-NH₂ was assembled on Fmoc deprotected [4-(2',4'-dimethoxyphenyl-Fmoc-aminomethyl)phenoxy] polystyrene resin using standard solid-phase synthesis procedures (DMF solvent, 20 % piperidine for Fmoc-deprotection) with 3 equivalents each of Fmoc-Lys(Boc)-OH, FmocCys(Trt)-OH and Fmoc∼Asp(OBut)OH, together with PyBOP, HOBt (3 equiv) and NMM (4.5 equiv). After the last acylation cycle, the peptidyl resin was washed with DMF and then treated repeatedly with excess iodine in MeOH to remove Cys(Trt) protecting groups and in order to effect dimerisation through disulphide bond formation. The peptidyl resin was washed successively with DMF and CH₂Cl₂ and was then treated for 1 h with CF₃COOH/CH₂Cl₂ (1:1) in order to remove the peptide from the resin and to remove the t-butyl protecting groups. Resin residue was then filtered off and the filtrate was rotary evaporated. After trituration with Et₂O, the product disulphide-(Fmoc-Asp-Cys-Lys-NH₂)₂, was filtered off and dried. An aliquot of this material (100 mg, 0.07 mmol) was dissolved in DMF (100 mL) and TBTU (100 mg, 0.31 mmol) and Prⁱ₂, NEt (0.1 mL, 0.5 mmol) were added. Product (disulphide-cyclo-(β-ε',β'-ε)-(Fmoc-Asp-Cys-Lys-NH₂)₂) precipitation was observed after ca. 30 min. After a total reaction time of 2 h, the precipitate was collected by centrifugation. It was then washed with Et₂O and dried. This material was Fmoc-deprotected by treatment with neat piperidine for 30 min. The resulting suspension was diluted with 10 volumes of Et₂O and the precipitate was collected by centrifugation. It was washed with Et₂O, dried and lyophilised from 0.1 % aq CF₃COOH. After redissolution in a minimum of the same solvent, the material was chromatographed on a RP-HPLC column (Vydac 218TP1022, 2.2 x 25 cm) at 10mL/min with a gradient from 0 to 10% CH₃CN in 0.1% aq CF₃COOH over 60 min. Pure title compound (15 mg, > 95% pure by analytical RP-HPLC at λ = 215 nm) was obtained from pooled selected fractions after lyophilisation. Amino acid analysis: Asp 2.0(2), Cys as cysteic acid 2.0(2), Lys 2.0(2). ES-MS :[M + H]⁺ = 689.7, [M + 2 H]²⁺ = 345.6; C₂₆H₄₄N₁₀O₈S₂ = 688.82.

The product was also characterised as the reduced form cyclo-(β-ε',β'-ε)-(H-Asp-Cys-Lys-NH₂)₂ as follows: An aliquot of the oxidised title compound was slurried in 0.1 M aq NaHCO₃ and 2 equivalents of DTT were added. After a few minutes dissolution was complete. RP-HPLC analysis (Vydac 218TP54, 0.46 x 25 cm, 1 mL/min, 0 to 12 % CH₃CN in 0.1% aq CF₃COOH over 20 min) showed complete conversion: t_{R} = 12 min (oxidised form), t_{R} = 7 min (reduced form). The reduced form was purified by preparative RP-HPLC. ES-MS: [M + H]⁺ = 691.6; C₂₆H₄₆N₁₀O₈S₂ = 690.83.

The reduced form of the peptide could be back-oxidised (air oxidation in 0.1 M aq NaHCO₃; 48 h) quantitatively to provide authentic title compound. Alternatively, the reduced form could be modified on both S atoms with Ellman's reagent (5,5'-dithio-bis-(2-nitrobenzoic acid)) as follows: The reduced peptide was dissolved in 0.1 M aq NaHCO₃ and was treated with an excess of Ellman's reagent in Me₂CO. The reaction was followed by analytical RP-HPLC (conditions as above except gradient from 0 to 30 % CH₃CN over 20 min), t_{R} (di-modified derivative) = 16 min. When the derivatisation reaction was complete, the product was purified by preparative RP-HPLC. ES-MS: [M + H]⁺ = 1085.6; C₄₀H₅₂N₁₂O₁₆S₄ = 1085.16.

### (Glp-Glu)₂-[disulphide-cyclo-(β-ε',β'-ε)-(Asp-Cys-Lys-NH₂)₂]

Reduced cyclo-(β-ε',β'-ε)-(H-Asp-Cys-Lys-NH₂)₂, dissolved in 0.1 % aq CF₃COOH, was mixed with excess Boc-Cys(Npys)-Resin (2g, 0.2 mmol). The pH of the suspension was raised to 5 by addition of 0.1 M aq NaHCO₃. The colour change in the suspension was indicative of Npys group liberation. The progress of the reaction was followed by RP-HPLC, after 18 h reaction time the soluble starting material had been consumed completely (without appearance of re-oxidised peptide), thus indicating complete resin attachment through disulphide formation between peptide and Boc-Cys residues anchored on the solid-phase. The peptidyl resin was then washed with water and DMF. N-terminal extension was then effected by reaction with Fmoc-Glu(OBut)-OH (425 mg, 1 mmol), TBTU (320 mg, 1 mmol) and Prⁱ₂NEt (0.4 mL, 2 mmol) in DMF for 1 h. After washing with DMF the Kaiser test indicated complete acylation. Fmoc-deprotection was then carried out with 20% piperidine in DMF (4 x 5 min). The resin was washed with DMF, CH₂Cl₂ and Et₂O and was dried. The next acylation was carried out with half of this material and Glp-OH (110 mg, 1mmol), together with TBTU (320 mg, 1 mmol) and Prⁱ₂NEt (0.2 mL, 1 mmol) for 1 h, when reaction was complete. After DMF and CH₂Cl₂ washes, the t-butyl protecting groups were then removed by treatment of the peptidyl resin with 95 % aq CF₃COOH for 30 min. It was then washed again with CH₂Cl₂ and Et₂O before being dried.

The fully assembled peptidyl resin was removed from the solid phase by twice treating with excess DTT in 0.1 M aq NH₄HCO₃. Resin residue was removed by filtration. The filtrate was lypohilised, redissolved in a minimum volume of 0.1 M aq NH₄KCO₃ and purified by preparative RP-HPLC (gradient elution in CF₃COOH/H₂O/CH₃CN solvent system) to yield 15 mg of pure reduced (Glp-Glu)₂[cyclo-(β-ε',β'-ε)-(Asp-Cys-Lys-NH₂)₂]. Analytical RP-HPLC (Vydac 218TP 54, 0.46 x 25 cm, 1 mL/min, gradient from 0 to 12% CH₃CN in 0.1% aq CF₃COOH over 20 min): t_{R}=10 min. ES-MS: [M+H]⁺=1171.6, [M+Na]⁺=1193.5, [M + 2H]²⁺ = 586.4; C₄₆H₁₀N₁₄O₁₈S₂ = 1171.26. Amino acid analysis: Asp 1.96(2), Glu (incl. Glp) 3.97(4), Cys (as cysteic acid) 2.09(2), Lys 1.99(2).

An aliquot (6 mg) of pure reduced (Glp-Glu)₂-[cyclo-(β-ε',β'-ε)-(Asp-Cys-Lys-NH₂)₂] was suspended in H₂O (2 mL) with stirring. After addition of concentrated ammonia solution (5 µL) the solution cleared. The reaction was monitored by analytical RP-HPLC, which indicated complete oxidation (with access to air oxygen) after 1 h. The product (Glp-Glu)₂-[disulphidecyclo(β-ε',β'-ε)-(Asp-Cys-Lys-NH₂)₂] was lyophilised. Analytical RP-HPLC (conditions as for reduced form): broad peak with t_{R} = 17 min. This material could be back-reduced quantitatively with DTT in 0.1 M aq NaHCO₃ to authentic (Glp-Glu)₂-[cyclo-(β-ε',β'-ε)-(Asp-Cys-Lys-NH₂)₂].

## Claims

1. Peptide compounds having cell proliferation regulatory activity of formula I, II or III: 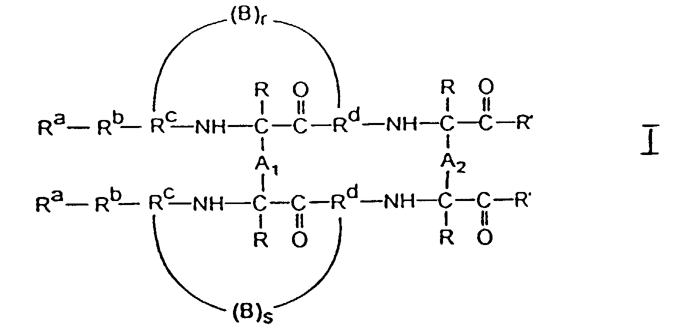 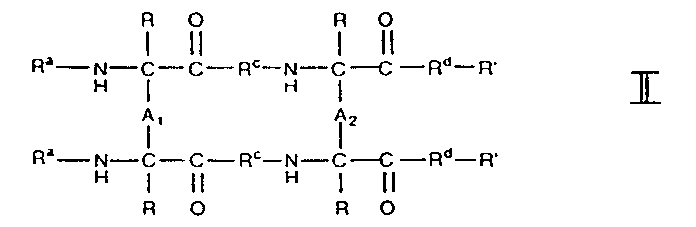 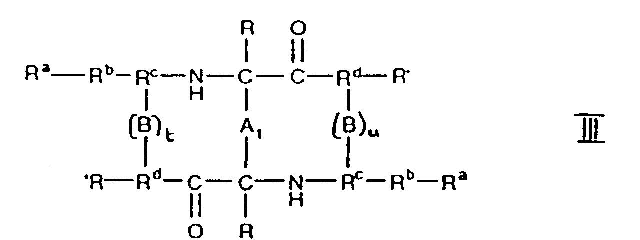 wherein
A₁ and A₂, which may be the same or different, represent
-(CH₂)ₘ- where m is 0 to 6,
-CH₂-S-S-CH₂-
-CH₂-CH=CH-CH₂-,
-CH₂-C≡C-CH₂-, 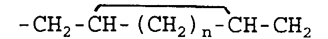 where n is 1 to 4, or 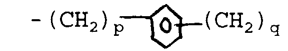
where p and q may each be 0 or 1; each R^{a} may independently be pyroglutamic acid (p-Glu), picolinic acid (Pic), 3-hydroxypicolinic acid (30H-Pic), proline, N-acetyl-proline, pyrazinecarboxylic acid, pyrazinedicarboxylic acid, nicotinic acid, isonicotinic acid, pipecolinic acid, pyrrolecarboxylic acid, anthranilic acid, quinolinecarboxylic acid, isoquinolinecarboxylic acid, furancarboxylic acid, orotic acid, or pyrazolecarboxylic acid;
each R^{b} may independently be serine (Ser), glutamic acid (Glu), aspartic acid (Asp), threonine (Thr) or allothreonine (aThr);
each R^{c} may independently be aspartic acid (Asp), glutamic acid (Glu) or cysteine (Cys);
each R^{d} may independently be lysine (Lys), ornithine (Orn) or cysteine (Cys);
r and s may each be 0 or 1;
t and u may each be 0 or 1 but at least one of t and u must be 1;
each group B represents the linked side chains of R^{c} and R^{d} such that
(a) each group B may independently represent -(CH₂)ᵥ-CONH-(CH₂)_{w}-, where v is 1 or 2 and w is 3 or 4, provided that in the peptide chain to which group B is attached R^{c} is Asp or Glu and R^{d} is Lys or Orn; or
(b) or, each group B may independently represent -CH₂-S-S-CH₂-, where R^{c} and R^{d} of the peptide chain to which group B is attached both represent Cys;
each R may be independently hydrogen or methyl; and
each R' may be a group -OH or -NH₂, and the salts thereof.

2. Peptide compounds as claimed in claim 1, wherein equivalent amino acid residues in each of the peptide chains have the same meaning.

3. Peptide compounds as claimed in claim 1 or claim 2, wherein the bridging moieties -A₁-, -A₂- and -B- have the same number of carbon atoms separating the two peptide chains.

4. Peptide compounds as claimed in any one of claims 1 to 3, wherein said bridging moieties -A₁-, -A₂- and -B- are ethylene or butylene.

5. Peptide compounds as claimed in any one of claims 1 to 4 of formula IV: 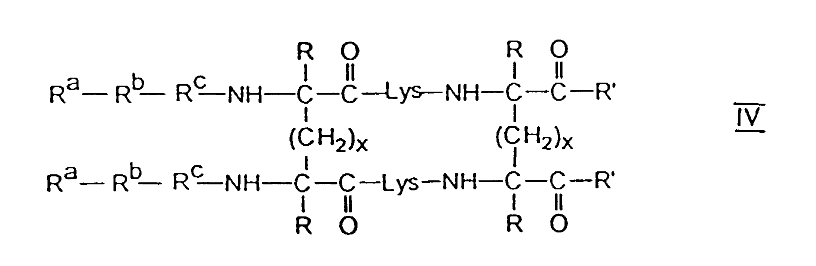 where both symbols x are either 2 or 4;
both groups R^{a}-R^{b}-R^{c}- are either pGlu-Glu-Asp- or Pic-Ser-Asp-, and each R¹ is a group -OH or -NH₂; and salts thereof.

6. Peptide compounds as claimed in claim 5 wherein x is 4.

7. Peptide compounds as claimed in claim 1 of formula V and VI:

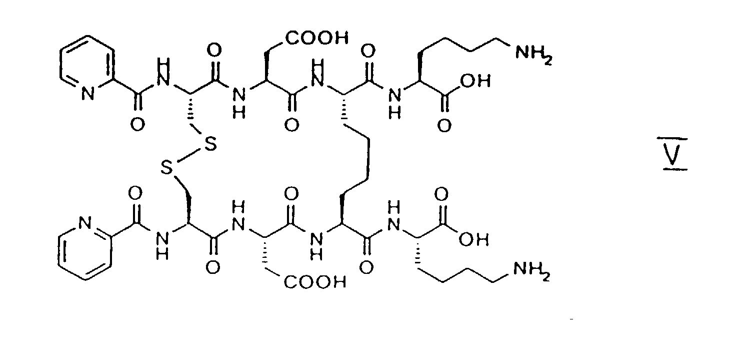

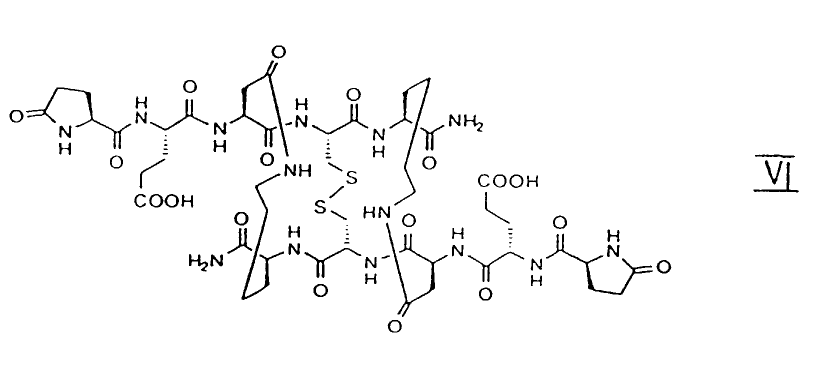

8. A pharmaceutical composition comprising a peptide compound as claimed in any one of claims 1 to 7 together with a pharmaceutical carrier or excipient.

9. Peptide compounds as claimed in any one of claims 1 to 7 for use in stimulating cell division.

10. Use of peptide compounds as claimed in any one of claims 1 to 7 in the manufacture of a medicament to stimulate cell division.

11. A process for preparing a peptide compound as claimed in any one of claims 1 to 7 comprising deprotecting a partially or fully protected derivative thereof.

12. A process for preparing a peptide compound as claimed in any one of claims 1 to 7, said process comprising
a) metallating and subsequently alkylating a bis-lactim ether to form a bis-lactim dipeptide ether;
b) hydrolysing the bis-lactim dipeptide ether of step (a) to form a bridged α,α'-diamino acid;
c) introducing the remaining amino acids including the other bridged amino acid in the peptide chains; and
d) deprotecting any protected group.

13. Compounds of formula X: 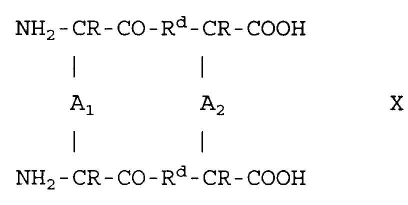 where A₁, A₂, R and R^{d} have the meanings given in claim 1.

14. Compounds of formula XIV: 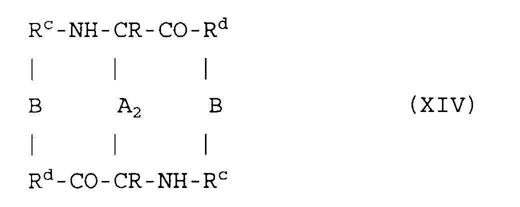 where R^{c}, R^{d}, R, A₂ and B have the meanings given in claim 1.

## Patentansprüche

1. Peptidverbindungen mit regulatorischer Aktivität auf die Zellproliferation der Formel I, II oder III: 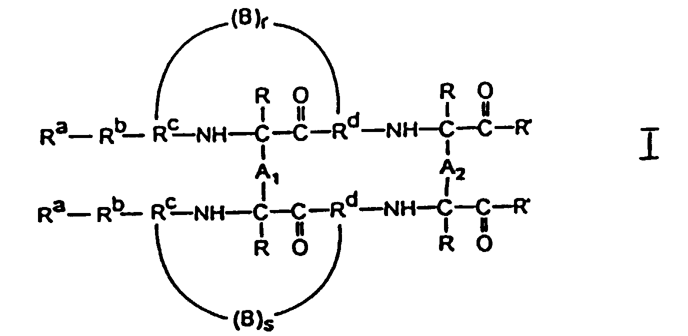 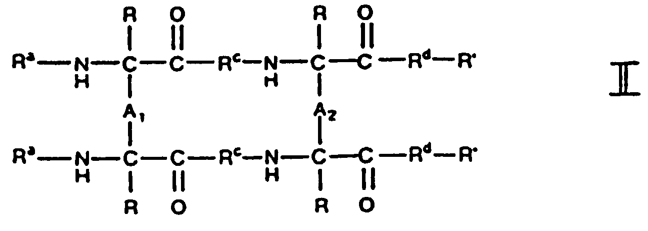 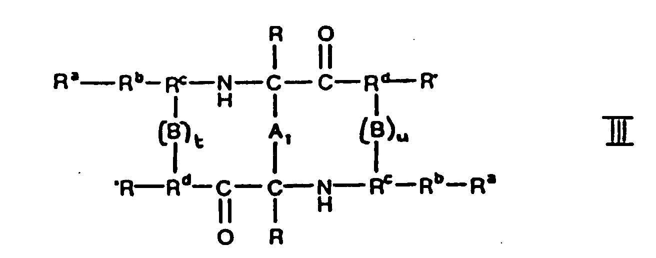 worin
A₁ und A₂, die gleich oder verschieden sein können, für
-(CH₂)ₘ-, worin m für 0-6 steht,
-CH₂-S-S-CH₂-
-CH₂-CH=CH-CH₂ -,
-CH₂-C≡C-CH₂, 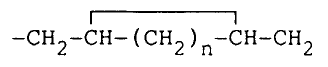 worin n für 1-4 steht, oder 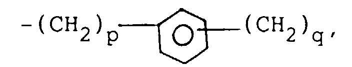 worin p und q jeweils 0 oder 1 sein können, stehen,
R^{a} jeweils unabhängig für Pyroglutaminsäure (p-Glu), Picolinsäure (Pic), 3-Hydroxypicolinsäure (3OH-Pic), Prolin, N-Acetylprolin, Pyrazincarbonsäure, Pyrazindicarbonsäure, Nicotinsäure, Isonicotinsäure, Pipecolinsäure, Pyrrolcarbonsäure, Anthranilsäure, Chinolincarbonsäure, Isochinolincarbonsäure, Furancarbonsäure, Orotsäure oder Pyrazolcarbonsäure stehen kann;
R^{b} jeweils unabhängig für Serin (Ser), Glutaminsäure (Glu), Asparaginsäure (Asp), Threonin (Thr) oder Allothreonin (aThr) stehen kann;
R^{c} jeweils unabhängig für Asparaginsäure (Asp), Glutaminsäure (Glu) oder Cystein (Cys) stehen kann;
R^{d} jeweils unabhängig für Lysin (Lys), Ornithin (Orn) oder Cystein (Cys) stehen kann;
r und s jeweils 0 oder 1 sein können;
t und u jeweils 0 oder 1 sein können, mit der Maßgabe, dass t und/oder u gleich 1 sein müssen;
die Gruppe B jeweils für die verknüpften Seitenketten von R^{c} und R^{d} steht, in einer Weise, dass
a) die Gruppe B jeweils unabhängig für -(CH₂)ᵥ-CONH-(CH₂)_{w}- steht, worin v für 1 oder 2 und w für 3 oder 4 steht, mit der Maßgabe, dass in der Peptidkette, an die die Gruppe B gebunden ist, R^{c} für Asp oder Glu und R^{d} für Lys oder Orn steht; oder
b) die Gruppe B jeweils unabhängig für -CH₂-S-S-CH₂- steht, worin sowohl R^{c} als auch R^{d} der Peptidkette, an die die Gruppe B gebunden ist, für Cys stehen;
R jeweils unabhängig für Wasserstoff oder Methyl stehen kann; und
R' jeweils für -OH oder -NH₂ stehen kann, und die Salze hiervon.

2. Peptidverbindungen nach Anspruch 1, worin äquivalente Aminosäurereste in jeder der Peptidketten die gleiche Bedeutung haben.

3. Peptidverbindungen nach Anspruch 1 oder 2, worin die verbrückenden Einheiten -A₁-, -A₂- und -B- die gleiche Anzahl Kohlenstoffatome aufweisen, die die zwei Peptidketten trennen.

4. Peptidverbindungen nach einem der Ansprüche 1 bis 3, worin es sich bei den verbrückenden Einheiten -A₁-, -A₂- und -B- um Ethylen oder Butylen handelt.

5. Peptidverbindungen nach einem der Ansprüche 1 bis 4, der Formel IV: 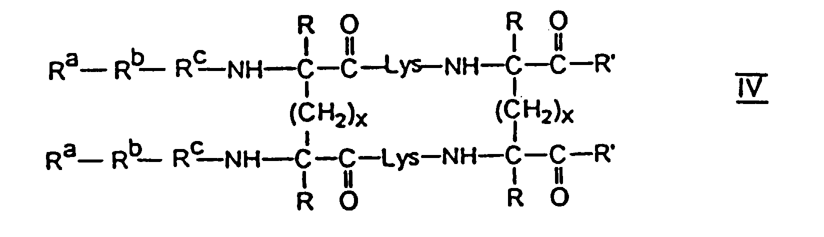 worin beide Symbole x entweder für 2 oder 4 stehen;
beide Gruppen R^{a}-R^{b}-R^{c}- entweder für pGlu-Glu-Asp- oder Pic-Ser-Asp-stehen und R¹ für eine -OH oder -NH₂-Gruppe steht; und
Salze davon.

6. Peptidverbindungen nach Anspruch 5, worin x für 4 steht.

7. Peptidverbindungen nach Anspruch 1, der Formel V und VI:

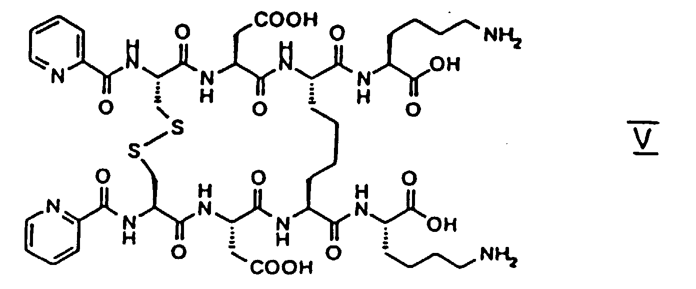

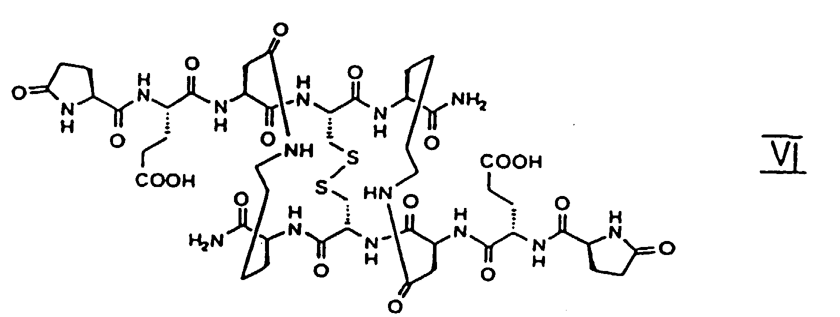

8. Pharmazeutische Zusammensetzung, umfassend eine Peptidverbindung nach einem der Ansprüche 1 bis 7 zusammen mit einem pharmazeutischen Träger oder Exzipienten.

9. Peptidverbindungen nach einem der Ansprüche 1 bis 7 zur Verwendung zur Stimulierung der Zellteilung.

10. Verwendung der Peptidverbindung nach einem der Ansprüche 1 bis 7 zur Herstellung eines Medikaments zur Stimulierung der Zellteilung.

11. Verfahren zur Herstellung einer Peptidverbindung nach einem der Ansprüche 1 bis 7, bei dem aus einem teilweise oder vollständig geschützten Derivat davon die Schutzgruppen abgespalten werden.

12. Verfahren zur Herstellung einer Peptidverbindung nach einem der Ansprüche 1 bis 7, bei dem
a) ein Bis-Lactimether unter Bildung eines Bis-Lactimdipeptidethers metalliert und anschließend alkyliert wird;
b) der Bis-Lactimdipeptidether aus Schritt a) unter Bildung einer verbrückten α,α'-Diaminosäure hydrolysiert wird;
c) die übrigen Aminosäuren einschließlich der anderen verbrückten Aminosäuren in die Peptidketten eingeführt werden; und
d) aus gegebenenfalls geschützten Gruppen die Schutzgruppen abgespalten werden.

13. Verbindungen der Formel X: 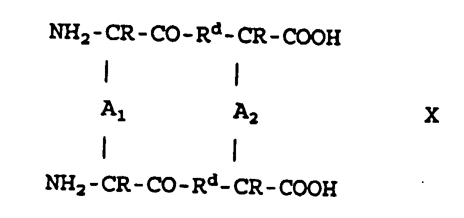 worin A₁, A₂, R und R^{d} die in Anspruch 1 angegebene Bedeutung haben.

14. Verbindungen der Formel XIV: 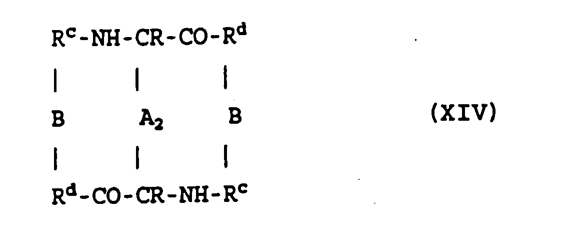 worin R^{c}, R^{d}, R, A₂ und B die in Anspruch 1 angegebene Bedeutung haben.

## Revendications

1. Composés peptidiques ayant une activité régulatrice de prolifération cellulaire, de formule I, II ou III : 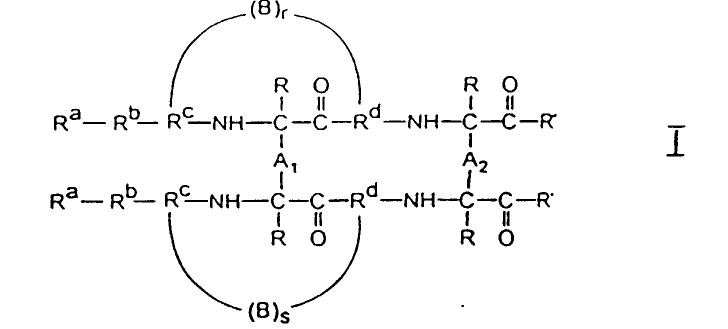 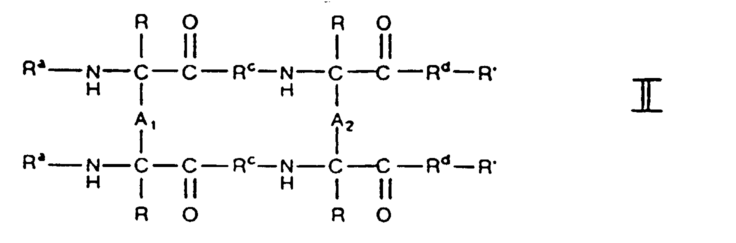 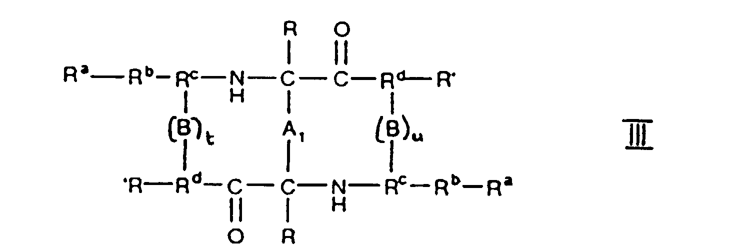 dans laquelle
A₁ et A₂, qui peuvent être identiques ou différents, représentent
-(CH₂)ₘ- où m est de 0 à 6,
-CH₂-S-S-CH₂-
-CH₂-CH=CH-CH₂-
-CH₂-C≡C-CH₂-, 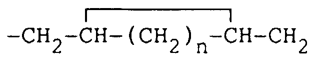 où n varie de 1 à 4, ou 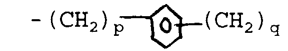 où p et q peuvent être chacun 0 ou 1 ;
chaque R^{a} peut être indépendamment l'acide pyroglutamique (p-Glu), l'acide picolinique (Pic), l'acide 3-hydroxypicolinique (3OH-Pic), la proline, la N-acétylproline, l'acide pyrazine-carboxylique, l'acide pyrazinedicarboxylique, l'acide nicotinique, l'acide isonicotinique, l'acide pipécolinique, l'acide pyrrole-carboxylique, l'acide anthranilique, l'acide quinoléine-carboxylique, l'acide isoquinoléine-carboxylique, l'acide furanne-carboxylique, l'acide orotique ou l'acide pyrazole-carboxylique ;
chaque R^{b} peut être indépendamment la sérine (Ser), l'acide glutamique (Glu), l'acide aspartique (Asp), la thréonine (Thr) ou l'allothréonine (aThr) ;
chaque R^{c} peut être indépendamment l'acide aspartique (Asp), l'acide glutamique (Glu) ou la cystéine (Cys) ;
chaque R^{d} peut être indépendamment la lysine (Lys), l'ornithine (Orn) ou la cystéine (Cys) ;
r et s peuvent être chacun 0 ou 1 ;
t et u peuvent être chacun 0 ou 1, mais au moins l'un de t et u doit être 1 ;
chaque groupe B représente les chaînes latérales reliées de R^{c} et R^{d}, en sorte que
(a) chaque groupe B peut représenter indépendamment -(CH₂)ᵥ-CONH-(CH₂)_{w}-, où v est 1 ou 2 et w est 3 ou 4, à condition que, dans la chaîne peptidique à laquelle est attaché le groupe B, R^{c} soit Asp ou Glu et R^{d} soit Lys ou Orn ; ou bien
(b) chaque groupe B peut représenter indépendamment -CH₂-S-S-CH₂-, lorsque R^{c} et R^{d} de la chaîne peptidique à laquelle est attaché le groupe B représentent tous deux Cys ;
chaque R peut être indépendamment un atome d'hydrogène ou un groupe méthyle ;
chaque R' peut être un groupe -OH ou -NH₂,
et leurs sels.

2. Composés peptidiques tels que revendiqués dans la revendication 1, dans lesquels des résidus d'acides aminés équivalents de chacune des chaînes peptidiques sont identiques.

3. Composés peptidiques tels que revendiqués dans la revendication 1 ou la revendication 2, dans lesquels les fragments pontants -A₁-, -A₂- et -B- ont le même nombre d'atomes de carbone séparant les deux chaînes peptidiques.

4. Composés peptidiques tels que revendiqués dans l'une quelconque des revendications 1 à 3, dans lesquels lesdits fragments pontants -A₁-, -A₂- et -B- sont des groupes éthylène ou butylène.

5. Composés peptidiques tels que revendiqués dans l'une quelconque des revendications 1 à 4, répondant à la formule IV : 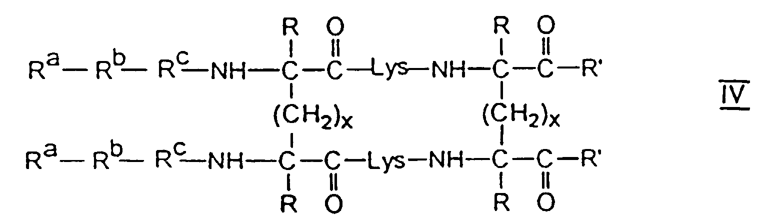 dans laquelle les deux symboles x représentent soit deux 2 ou 4,
les deux groupes R^{a}-R^{b}-R^{c} sont soit pGlu-Glu-Asp-ou Pic-Ser-Asp-, et chaque R¹ est un groupe -OH ou -NH₂ ;
et leurs sels.

6. Composés peptidiques tels que revendiqués dans la revendication 5, dans lesquels x est 4.

7. Composés peptidiques tels que revendiqués dans la revendication 1, répondant à la formule V ou VI :

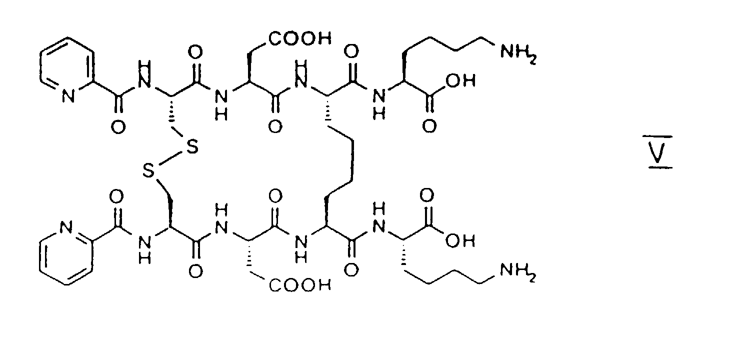

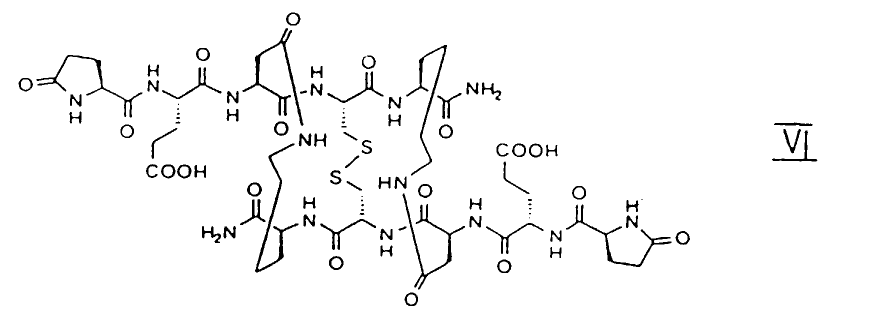

8. Composition pharmaceutique comprenant un composé peptidique tel que revendiqué dans l'une quelconque des revendications 1 à 7 ainsi qu'un vehicule ou excipient pharmaceutique.

9. Composés peptidiques tels que revendiqués dans l'une quelconque des revendications 1 à 7, pour une utilisation dans la stimulation de la division cellulaire.

10. Utilisation de composés peptidiques tels que revendiqués dans l'une quelconque des revendications 1 à 7, pour la fabrication d'un médicament destiné à stimuler la division cellulaire.

11. Procédé pour préparer un composé peptidique tel que revendiqué dans l'une quelconque des revendications 1 à 7, comprenant la déprotection d'un dérivé partiellement ou totalement protégé de celui-ci.

12. Procédé pour préparer un composé peptidique tel que revendiqué dans l'une quelconque des revendications 1 à 7, ledit procédé consistant à :
a) métaller et ensuite alkyler un éther de bis-lactime pour former un éther dipeptidique de bis-lactime ;
b) hydrolyser l'éther dipeptidique de bis-lactime de l'étape (a) pour former un acide α,α'-diaminé ponté ;
c) introduire les acides aminés restants, y compris l'autre acide aminé ponté, des chaînes peptidiques ; et
d) la déprotection de tout groupe protégé.

13. Composés de formule X : 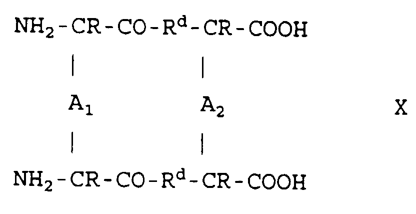 où A₁, A₂, R et R^{d} sont tels que définis dans la revendication 1.

14. Composés de formule XIV : 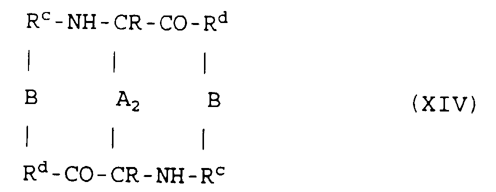 où R^{c}, R^{d}, R, A₂ et B sont tels que définis dans la revendication 1.
